# EUROPÄISCHE PATENTANMELDUNG

(11) **EP 1 026 138 A1**
(43) Veröffentlichungstag der Anmeldung: **09.08.2000**
(21) Anmeldenummer: 99123815.5
(22) Anmeldetag: 01.12.1999
(51) Int. Cl.: C07C 17/02, C07C 17/383, C07C 19/045

(54) **Verfahren zur Herstellung von 1,2-Dichlorethan**

(30) Priorität: 06.02.1999 DE 19904836
(71) Anmelder: Krupp Uhde GmbH, 44141 Dortmund (DE)
(72) Erfinder: Motz, Joachim, Dr., 65779 Kelkheim (DE)
(74) Vertreter: Patentanwälte Meinke, Dabringhaus und Partner

(57) **Zusammenfassung**

Bei einem Verfahren zur Herstellung von 1,2-Dichlorethan (EDC) durch Reaktion von Ethylen und Chlor in der Flüssigphase (Direktchlorierung) in Gegenwart eines Katalysators, wobei das produzierte 1,2-Dichlorethan in der Gasphase abgezogen wird, wobei aus dem produzierten 1,2-Dichlorethan in einer Hochsiederkolonne und anschließend in einer Vakuumkolonne Hochsieder abgetrennt werden und auβerdem aus dem produzierten 1,2-Dichlorethan Leichtsieder und Gase in einer EDC-Stripperkolonne oder in einer Leichtsiederkolonne abgetrennt werden, soll eine großtechnisch realisierbare Möglichkeit geschaffen werden, den entstehenden Chlorwasserstoff am Kopf der EDC-Stripper- oder Leichtsiederkolonne wirkungsvoll zu entfernen, um dort Korrosion zu vermeiden.

Dies wird dadurch erreicht, daß das aus der Hochsiederkolonne austretende, gereinigte 1,2-Dichlorethan in der EDC-Stripperkolonne oder in der Leichtsiederkolonne unter Abtrennung von Leichtsiedern und Gasen gereinigt wird, indem die aus der EDC-Stripper- oder Leichtsiederkolonne austretenden Brüden oder/und kondensierten Brüden mit einer basisch wirkenden Substanz neutralisiert werden und damit der Chlorwasserstoff entfernt wird.

Hierzu ist die einzige Figur zu veröffentlichen.

## Beschreibung

Die Erfindung betrifft ein Verfahren zur Herstellung von 1,2-Dichlorethan (EDC) durch Reaktion von Ethylen und Chlor in der Flüssigphase (Direktchlorierung) in Gegenwart eines Katalysators, wobei das produzierte 1,2-Dichlorethan in der Gasphase abgezogen wird, wobei aus dem produzierten 1,2-Dichlorethan in einer Hochsiederkolonne und anschließend in einer Vakuumkolonne Hochsieder abgetrennt werden und außerdem aus dem produzierten 1,2-Dichlorethan Leichtsieder und Gase in einer EDC-Stripperkolonne oder in einer Leichtsiederkolonne abgetrennt werden.

Großtechnisch wird 1,2-Dichlorethan (EDC) durch Direktchlorierung von Ethylen mit Chlorgas hergestellt. Diese Reaktion findet unter der Einwirkung eines oder mehrerer Katalysatoren und/oder Promotoren in der Flüssigphase bei Temperaturen von 75°C bis 100°C und Drücken von 0,8 bar absolut bis 3 bar absolut statt. Dabei wird das produzierte 1,2-Dichlorethan dampfförmig abgezogen. Die entstehende Reaktionsenthalpie kann durch Sieden und Kondensieren des 1,2-Dichlorethans und Rückführung des um den Produktabzug verringerten kondensierten 1,2-Dichlorethans abgeführt werden. Alternativ ist es auch möglich, durch ausreichende Kühlung des siedenden flüssigen 1,2-Dichlorethans nur die gewünschte Menge an produziertem 1,2-Dichlorethan zu verdampfen. Die Kühlung erfolgt dabei idealerweise durch Kühlung der Flüssigphase, die in einem Zwangskreislauf oder Naturumlauf zirkuliert. Ferner ist es möglich, den Druck im Reaktor so einzustellen, daß dort keine Verdampfung des 1,2-Dichlorethans auftritt, sondern das produzierte 1,2-Dichlorethan durch Entspannung und damit einhergehende partielle Verdampfung zirkulierenden flüssigen 1,2-Dichlorethans abgezogen wird. Auch bei dieser Verfahrensführung erfolgt die Kühlung der Flüssigphase in einem Zwangskreislauf oder Naturumlauf. Das abgezogene produzierte 1,2-Dichlorethan wird unter Abtrennung von Hochsiedern in einer Hochsiederkolonne und einer Vakuumkolonne und Abtrennung von Leichtsiedern und Gasen, wie Ethylen und Chlorwasserstoff, in einer EDC-Stripperkolonne gereinigt. Die bei der Reinigung anfallenden Nebenprodukte werden verbrannt, wobei Chlorwasserstoff aus den Rauchgasen zurückgewonnen wird.

Es gibt zwar Verfahren dieser Art, bei denen Gase und Leichtsieder in einer Leichtsiederkolonne abgetrennt werden. Wegen der im Kolonnenkopf, Kopfkondensator, Rücklaufbehälter und der Rücklaufpumpe auftretenden Aufkonzentrierung von Wasser und Chlorwasserstoff kommt es jedoch zu erheblichen Korrosionsproblemen, die die Anwendung extrem teurer Konstruktionsmaterialien erfordern, wodurch jedoch die Bildung von FeCl₃ nicht ganz vermieden werden kann. Verfahren zur Trocknung des Kolonnenbrüdens sind noch nicht ausgereift und funktionieren großtechnisch nicht zufriedenstellend.

Erhöhte Konzentration an FeCl₃ in 1,2-Dichlorethan führen zu verstärkter Verkokung des Spaltofens und erheblicher Verkürzung der Standzeit bei der thermischen Spaltung von 1,2-Dichlorethan zu Vinylchlorid. Wirtschaftlichen Zwängen folgend, wonach Betreiber von Spaltöfen Standzeiten von über einem Jahr fordern, muß die Konzentration an Fe³⁺ in 1,2-Dichlorethan unter 0,5 ppm liegen.

Aufgabe der Erfindung ist es daher, bei einem gattungsgemäßen Verfahren eine großtechnisch realisierbare Möglichkeit zu schaffen, den entstehenden Chlorwasserstoff am Kopf der EDC-Stripper- oder Leichtsiederkolonne wirkungsvoll zu entfernen, um dort Korrosion zu vermeiden und die Spezifikation der Spaltofenbetreiber für 1,2-Dichlorethan einhalten zu können.

Diese Aufgabe wird bei einem Verfahren der eingangs bezeichneten Art erfindungsgemäß dadurch gelöst, daß das aus der Hochsiederkolonne austretende, gereinigte 1,2-Dichlorethan in der EDC-Stripperkolonne oder in der Leichtsiederkolonne unter Abtrennung von Leichtsiedern und Gasen gereinigt wird, indem die aus der EDC-Stripper- oder Leichtsiederkolonne austretenden Brüden oder/und kondensierten Brüden mit einer basisch wirkenden Substanz neutralisiert werden und damit der Chlorwasserstoff entfernt wird.

Durch diese Verfahrensführung ist es auch in großtechnischem Maßstab möglich, den Chlorwasserstoff am Kopf der Kolonne zu neutralisieren und damit wirkungsvoll zu entfernen. Dadurch wird eine Korrosion vermieden und die Spezifikation der Spaltofenbetreiber für 1,2-Dichlorethan kann zuverlässig eingehalten werden. Der Aufwand für diese Verfahrensführung ist dabei verhältnismäßig gering, so daß sich dieses Verfahren auch im großtechnischen Maßstab wirtschaftlich durchführen läßt.

Bevorzugt ist vorgesehen, daß als basisch wirkende Substanz Natronlauge eingesetzt wird.

In weiterer vorteilhafter Ausgestaltung ist vorgesehen, daß die neutralisierten Brüden anschließend auskondensiert und dann in eine wässrige und in eine organische Phase getrennt werden und die organische Phase in die EDC-Kolonne zurückgeführt wird. Die organische Phase kann dann in der EDC-Kolonne weiter behandelt werden.

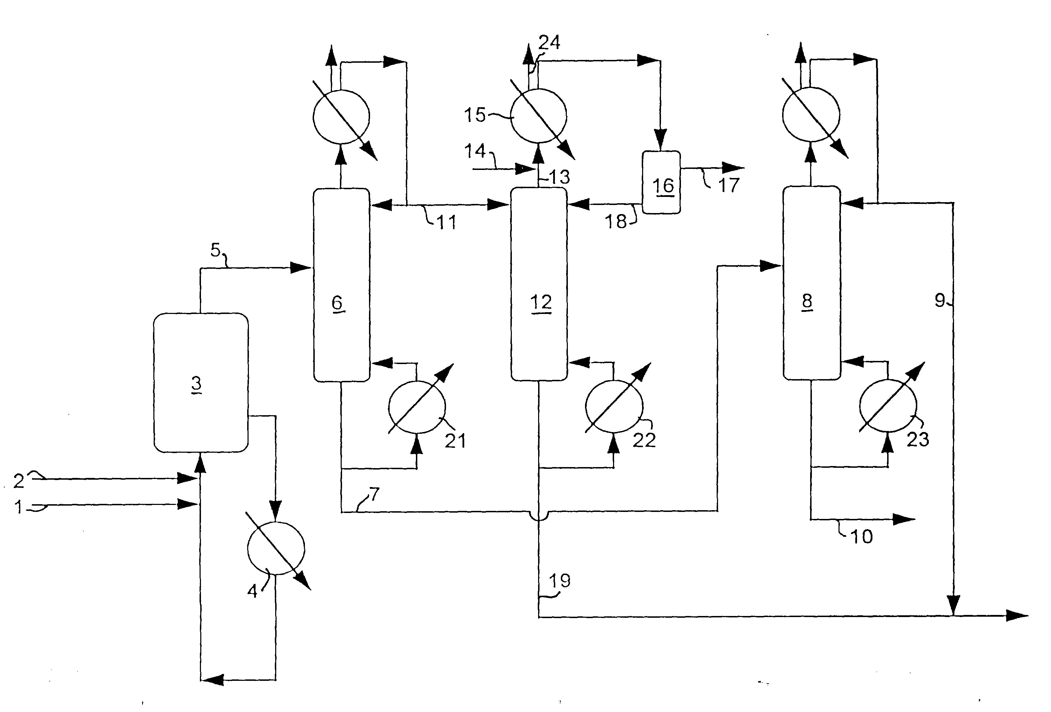

Die Erfindung ist nachstehend anhand der Zeichnung beispielsweise näher erläutert. Diese zeigt in der einzigen Figur ein Verfahrensfließbild, das den erfindungsgemäßen Verfahrensablauf beispielhaft zeigt.

Eine Anlage zur Durchführung des erfindungsgemäßen Verfahrens zur Herstellung von 1,2-Dichlorethan weist zunächst einen Direktchlorierungsreaktor 3 auf, in den über eine Leitung 1 Chlor und eine Leitung 2 Ethylen in flüssiger Form zugeleitet werden. Im Reaktor 3 wird aus Chlor und Ethylen bei Temperaturen von 75°C bis 100°C und Drükken von 0,8 bar absolut bis 3 bar absolut unter Zusatz von einem oder mehreren Katalysatoren und/oder Promotoren 1,2-Dichlorethan erzeugt. Die dabei entstehende Reaktionswärme wird über einen Wärmeaustauscher 4 so weit abgezogen, daß lediglich die produzierte Menge an 1,2-Dichlorethan über eine Leitung 5 in eine Hochsiederkolonne 6 verdampft wird.

Der Blasenabzug 7 der Hochsiederkolonne 6 enthält 3 bis 10 % Hochsieder, die über den Blasenabzug 7 in eine Vakuumkolonne 8 geleitet werden. In der Vakuumkolonne 8 werden die Hochsieder weiter aufkonzentriert und 1,2-Dichlorethan am Kopf der Vakuumkolonne 8 zurückgewonnen und über eine Leitung 9 abgeführt. Die aufkonzentrierten Hochsieder werden über eine Leitung 10 zur Verbrennung und Rückgewinnung von Chlorwasserstoff ausgeschleust.

Am Kopf der Hochsiederkolonne 6 werden die entstandenen Brüden über einen Brüdenabzug 11 abgezogen und in eine EDC-Stripperkolonne 12 geleitet. In dieser EDC-Stripperkolonne 12 wird das 1,2-Dichlorethan in gereinigter Form am Fuß abgezogen und über einen Blasenabzug 19 abgeleitet, das gereinigte 1,2-Dichlorethan ist somit der Blasenabzug der EDC-Stripperkolonne gemeinsam mit dem Brüdenabzug der Vakuumkolonne 8, deren beide Ableitungen 19 und 9 dementsprechend zusammengeführt werden.

Erfindungswesentlich werden die in der EDC-Stripperkolonne 12 entstehenden Brüden am Kopf über einen Brüdenabzug 13 abgeführt und durch Zufügung von Natronlauge über eine Zuführleitung 14 neutralisiert. Die so neutralisierten Brüden werden anschließend in einem Kondensator 15 auskondensiert und gelangen dann in einen Rückflußbehälter 16. In diesem Rückflußbehälter 16 wird die wässrige Phase dekantiert, der entsprechende Abfluß ist mit 17 bezeichnet. Die organische Phase gelangt über eine Rückflußleitung 18 zurück in die EDC-Stripperkolonne 12.

Die Beheizung der einzelnen Kolonnenverdampfer 21, 22, 23 kann auch durch Nutzung der Reaktionswärme aus der Direktchlorierung im Reaktor 3 erfolgen. Es versteht sich von selbst, daß die vorbeschriebene Ausführungsform einer Anlage zur Durchführung des Verfahrens modifiziert werden kann, ohne den Erfindungsgedanken zu verlassen.

Das folgende Beispiel dient der weiteren Erläuterung der Erfindung. Alle Temperaturen verstehen sich in Celsius-Graden, alle Druckangaben als Absolutdrucke. Gew.-ppm bedeuten mg einer Substanz pro kg Gesamtgewicht der 1,2-Dichlorethan-Fraktion.

### Beispiel

Die EDC-Stripperkolonne 12 wurde durch Brüdenabzug der Hochsiederkolonne 6 mit einem Massenstrom von 63.956 kg/h folgender Zusammensetzung beschickt:

| | |
|---|---|
| 99,95 Gew.-% | 1,2-Dichlorethan |
| 150 Gew.-ppm | Chlorwasserstoff |
| 45 Gew.-ppm | Ethylen |
| 100 Gew.-ppm | 1,1-Dichlorethan |
| 125 Gew.-ppm | 1,1,2-Trichlorethan |
| 15 Gew.-ppm | Stickstoff |
| 8 Gew.-ppm | Wasser |
| 8 Gew.-ppm | Sauerstoff |
| 0,5 Gew.-ppm | Fe³⁺ |

Im Gleichgewichtszustand der EDC-Stripperkolonne 12 stellte sich durch kontinuierlichen Abzug von 63.936 kg/h Sumpfprodukt in der Blase der EDC-Stripperkolonne 12 bei einem Druck von 1,85 bar eine Temperatur von 108°C ein. Am Kopf der Kolonne wurden bei einem Druck von 1,5 bar und bei Sattdampftemperatur von 98,6°C eine Menge von 16.210 kg/h Brüden mit 58 kg/h Natronlauge vermischt, deren Konzentration an Natriumhydroxid bei 20 Gew.-% lag. Im Wärmeaustausch mit Kühlwasser und Kältemittel im Kondensator 15 kondensierte der Brüden und floß mit einer Temperatur von 45°C in den Rückflußbehälter 16 ab. 73 kg/h wässrige Phase wurden dekantiert, die organische Phase wurde über den Rücklauf 18 in die Kolonne zurückgeleitet. Zur Druckhaltung wurden 5 kg/h Gase und nicht kondensierte Dämpfe über eine Ableitung 24 ausgeschleust (siehe Figur).

Der Blasenabzug 19 der EDC-Stripperkolonne hatte folgende Zusammensetzung:

| | |
|---|---|
| 99,98 Gew.-% | 1,2-Dichlorethan |
| 0 Gew.-ppm | Chlorwasserstoff |
| 0 Gew.-ppm | Ethylen |
| 55 Gew.-ppm | 1,1-Dichlorethan |
| 125 Gew.-ppm | 1,1,2-Trichlorethan |
| 0 Gew.-ppm | Stickstoff |
| 3 Gew.-ppm | Wasser |
| 0,5 Gew.-ppm | Fe³⁺. |

Es versteht sich von selbst, daß vorstehendes Ausführungsbeispiel nur zur Verdeutlichung der Erfindung dient, selbstverständlich ist das erfindungsgemäße Verfahren auch für andere Verfahrensbedingungen geeignet.

## Patentansprüche

1. Verfahren zur Herstellung von 1,2-Dichlorethan (EDC) durch Reaktion von Ethylen und Chlor in der Flüssigphase (Direktchlorierung) in Gegenwart eines Katalysators, wobei das produzierte 1,2-Dichlorethan in der Gasphase abgezogen wird, wobei aus dem produzierten 1,2-Dichlorethan in einer Hochsiederkolonne und anschließend in einer Vakuumkolonne Hochsieder abgetrennt werden und außerdem aus dem produzierten 1,2-Dichlorethan Leichtsieder und Gase in einer EDC-Stripperkolonne oder in einer Leichtsiederkolonne abgetrennt werden,
dadurch gekennzeichnet,
daß das aus der Hochsiederkolonne austretende, gereinigte 1,2-Dichlorethan in der EDC-Stripperkolonne oder in der Leichtsiederkolonne unter Abtrennung von Leichtsiedern und Gasen gereinigt wird, indem die aus der EDC-Stripper- oder Leichtsiederkolonne austretenden Brüden oder/und kondensierten Brüden mit einer basisch wirkenden Substanz neutralisiert werden und damit der Chlorwasserstoff entfernt wird.

2. Verfahren nach Anspruch 1,
dadurch gekennzeichnet,
daß als basisch wirkende Substanz Natronlauge eingesetzt wird.

3. Verfahren nach Anspruch 1 oder 2,
dadurch gekennzeichnet,
daß die neutralisierten Brüden anschließend auskondensiert und dann in eine wässrige und in eine organische Phase getrennt werden und die organische Phase in die EDC-Kolonne zurückgeführt wird.
